# EUROPÄISCHE PATENTANMELDUNG

(11) **EP 0 653 487 A1**
(43) Veröffentlichungstag der Anmeldung: **17.05.1995**
(21) Anmeldenummer: 93118035.0
(22) Anmeldetag: 07.11.1993
(51) Int. Cl.: C12N 5/02, C12N 5/08

(54) **Serum- und proteinfrei wachsende Zellen**

(71) Anmelder: Messi, Ferruccio, Dr., CH-8003 Zürich (CH)
(72) Erfinder: Messi, Ferruccio, Dr., CH-8003 Zürich (CH)
(74) Vertreter: Blum, Rudolf Emil Ernst

(57) **Zusammenfassung**

Es werden substrat-, serum- und proteinunabhängige eukaryontische Zellen beschrieben, die als Wirtszellen für die Gen- und Biotechnologie speziell geeignet sind und nie einem speziell mutagen wirkenden Stoff ausgesetzt worden sind. Ferner wird ein Verfahren zu deren Herstellung beschrieben.

## Beschreibung

Die vorliegende Erfindung betrifft serum- und proteinfrei wachsende Zellen, insbesondere CHO (Chinese Hamster Ovary)-Zellen, d.h. Zellen, die aus den Ovarien des chinesischen Hamsters gewonnen wurden, sowie ein Verfahren zu deren Selektion.

Als Wirtszellen für Klonierungen weisen einzellige Organismen wie Bakterien, Hefen und Pilze grosse Vorteile auf, da sie eine relativ stabile Hülle aufweisen, nicht von einem festen Substrat abhängig und in definierbaren Kulturmedien mit guten Generierungszeiten vermehrungsfähig sind.

Nachteil bei der Verwendung solcher einzelliger Organismen als Wirtszellen ist, dass die in ihnen hergestellten Expressionsprodukte oft eine falsche dreidimensionale Struktur aufweisen und dass solche Organismen nur selten in der Lage sind posttranslationale Modifikationen durchzuführen.

Für viele Anwendungen ist es deshalb unerlässlich, als Expressionssystem transformierte Säugerzellen zu verwenden.

Ein grosser Nachteil der heute gebräuchlichen Zellen ist, dass viele nur auf einem festen Substrat gezüchtet werden können, und dass auch submers profilierfähige Zellen in einem serum- und proteinhaltigen und damit chemisch undefinierten Medium gezüchtet werden müssen.

Die Substratabhängigkeit führt zu einer relativ geringen Zelltrockenmasse von üblicherweise 0,5-1 g/l, sowie einer limitierten Durchmischung im Reaktor und damit ungleichmässigem Nährstoffzugriff. Ferner ist die Zusammensetzung von serum- und proteinhaltigen Kulturmedien schlecht oder gar nicht kontrollierbar, da das Serum tierischer Herkunft ist, was einer vollsynthetischen und damit chemisch definierten Herstellung entgegensteht. Die tierische Herkunft des Serums hat aber nicht nur den Nachteil, dass dieses keine genau definiertbare Zusammensetzung hat, sondern auch den einer stark erhöhten Kontaminationsgefahr, beispielsweise durch Brione oder Viren. Ein weiterer Nachteil dieser serum- und proteinhaltigen Medien sind deren hohe Kosten, wobei der Serum- resp. Protein-Anteil ca. 90% dieser Kosten ausmachen. Ausserdem sind die darin enthaltenen tierischen Extrakte, heute aus politischen Gründen nicht mehr willkommen.

Ein weiterer Nachteil dieser serum- und proteinhaltigen Kulturmedien ist die problematische, aufwendige Reinigung des Expressionsprodukts.

Eine Zelle, die sich substrat-, serum- und/oder proteinunabhängig vermehrt, wurde bereits 1990 von S. Kurano et al. in Cytotechnology 4, Seite 243-250 erwähnt. Die Herstellung solcher Zellen wurde von F. Messi in "Tuning of structure and function of Chinese Hamster Ovary (CHO) cells by systematic design of defined micro-environments", Diss. ETH Nr. 9559 (1991) erstmals beschrieben. Die dort geoffenbarten Zellen haben gewissen Eigenschaften die denjenigen von Krebszellen ähnlich sind. Sie sind aus der CHO-DUKXB1 Zellinie entwickelt worden. Die CHO-DUKXB1- Zellen nach Urlaub und Chasin (G. Urlaub und L.A. Chasin, Proc.Acad.Sci. USA 77, 4216-4220 (1980)) sind durch mutagene Behandlung mit Ethylmethansulfonat aus CHO-K1-Zellen nach Kao und Puck (F.T. Kao und T.T. Puck, Proc.Acad.Sci USA 60, 1275-1281 (1968)) hervorgegangen.

Es ist schon lange bekannt, dass die Behandlung von CHO-Zellen mit Ethylmethansulfonat nicht nur das DHFR-Gen beeinflusst, sondern auch andere Mutationen hervorrufen kann. Kürzlich wurde nun nachgewiesen, dass Behandlung mit Ethylmethansulfonat die Anzahl serum- und proteinfrei lebensfähiger Zellen aus einer CHO-DUKXB1-Kultur sowie deren Wachstumsgeschwindigkeit positiv beeinflusst (Christine R. Gandor, Diss. ETH Nr. 10087 (1993)).

Die oben beschriebenen serum- und proteinfrei wachsenden Zellen haben gegenüber den heute gebräuchlichen Zellen den Vorteil, dass sie unabhängig von Serum und Protein in einem vollsynthetischen und deshalb volldefinierten Medium mit gut reproduzierbarer Qualität gezüchtet werden können. Da im Kulturmedium für diese Zellen auf tierische Extrakte verzichtet werden kann, ist die Kontaminationsgefahr geringer und die Reinigung eines mittels dieser Zellen exprimierten Produktes einfacher. Durch die Substratunabhängigkeit ergibt sich als weiterer Vorteil die Möglichkeit der ständigen Ueberprüfung der Zellqualität und die Möglichkeit der leichten Isolierbarkeit zellinterner Bestandteile, die beispielsweise bei Kulturen auf Mikroträgern (microcarriers) und insbesondere bei Kulturen in Hohlfasern kaum möglich ist. Ein weiterer Vorteil ist die gute Durchmischung im Reaktor durch die, im Gegensatz zu mit Hohlfasern gefüllten Patronen, ein Konzentrationsgradient der Mediumbestandteile drastisch vermindert oder verhindert wird.

Nachteil dieser bisher bekannten substrat-, serum- und proteinunabhängig wachsender Zellen ist, dass sie von der CHO-DUKXB1-Zelle abstammen und somit allen das Dehydrofolatreduktase (DHFR)-Gen fehlt (CHO-DHFR⁻). Sie können deshalb nur in Gegenwart von Hypoxanthin, Glycin und Thymidin gezüchtet werden. Hypoxanthin und Thymidin beeinträchtigen aber die Lagerstabilität eines protein- und serumfreien Kulturmediums.

Ferner hat sich das Fehlen des rezessiven DHFR-Gens generell als unstabiler Marker erwiesen, da die Zellen dazu neigen zu revertieren und dadurch beim Klonieren häufig falsche positive Klone ausweisen.

Ausserdem ist nicht auszuschliessen, dass neben der Beeinträchtigung des DHFR-Gens infolge der mutagenen Bedingungen, denen die Zellinie ausgesetzt war, auch noch Mutationen an anderen Stellen des Genoms erzeugt worden sind.

Ziel der vorliegenden Erfindung war es deshalb, eine eukaryontische Zelle, insbesondere eine CHO-Zelle bereitzustellen, die substratunabhängig ist und in einem serum- und proteinfreien, einfachen, vollsynthetischen Kulturmedium wächst, und die nie speziell mutagenen Bedingungen ausgesetzt war und das DHFR-Gen enthält.

Diese Aufgabe wird gelöst durch das Bereitstellen einer eukaryontischen Zelle mit im wesentlichen runder Morphologie, die substratunabhängig ist und in einem serum- und proteinfreien Medium gezüchtet werden kann und die dadurch gekennzeichnet ist, dass sie in einem Medium kultivierbar ist, welches keine Stoffe enthält, die die ursprüngliche Zelle in ihrer natürlichen Umgebung selbst produzieren kann (Minimalmedium).

Bevorzugte Ausführungsarten sind den abhängigen Ansprüchen zu entnehmen. Ueberraschend wurde gefunden, dass auch aus Zellen, die nie speziell mutagenen Bedingungen ausgesetzt worden sind, einzelne Zellen in serum- und/oder proteinfreiem Kulturmedium lebensfähig sind. Solche Zellen wurden mittels des Verfahrens, das ebenfalls Gegenstand dieser Erfindung ist, isoliert und gezüchtet. Dabei wurde festgestellt, dass beispielsweise CHO-Zellen, die aus der Zelllinie CHO-K1, dem nicht mit einem Mutagen behandelten Vorläufer der CHO-DUKXB1-Zelllinie, hervorgegangen sind, gleich gute oder bessere Wachstumseigenschaften aufweisen, wie die im Stand der Technik beschriebenen Zellen.

Ein Vorteil dieser CHO-Zellen ist, dass sie in einem vollsynthetischen thymidin-, hypoxanthin- und glycinfreien Medium gezüchtet werden können.

Allerdings hat sich gezeigt, dass die Zugabe dieser Stoffe die Wachstumsgeschwindigkeit der Zelle in einem gegebenen Medium positiv beeinflussen kann.

Ob die Zelle bevorzugt in einem thymidin-, hypoxanthin- und glycinfreien oder diese Stoffe enthaltenden Medium kultiviert werden soll, ist von Fall zu Fall zu entscheiden.

Solche Zellen können hergestellt werden, indem sie aus dem serumhaltigen Kulturmedium in ein serumfreies Medium gebracht werden. Bei diesem Verfahren sterben die meisten Zellen infolge des schlagartigen Serumentzuges innerhalb von einigen Tagen ab und nur wenige überleben. Diese überlebenden Zellen werden ohne Verdünnung kultiviert, bis ein lebensfähiges Inoculum sichergestellt ist. Da die Zellen bei ihrer Vermehrung Glucose in Lactat verwandeln, fällt der pH innerhalb weniger Tage unter den für das Wachstum günstigen Wert. Deshalb wird in Abständen von einigen Tagen ein Teil, üblicherweise die Hälfte des flüssigen Kulturmediums, durch frisches Kulturmedium ersetzt. Derart kann der pH im für die Zellvermehrung physiologisch günstigen Bereich von üblicherweise pH 7.0 - 7.6 gehalten werden. Zudem werden andere für die Zellen lebenswichtige und/oder vermehrungsfördernde Stoffe ersetzt.

Je nach Art des verwendeten Kulturmediums resp. der verwendeten Zellen muss die zellhaltige Kulturlösung vor dem Ersetzen von Kulturmedium zentrifugiert werden.

Die ursprünglich auf einem Substrat fixierten, eukaryontischen Zellen gehen in einem serum- und proteinfreien Medium in Suspension, da spezielle Proteine, die die Haftung am Substrat begünstigen, fehlen.

Im Kulturmedium, das in den Verfahren des Standes der Technik verwendet wurde, ist der Anteil an überlebenden, nicht abermals einem Mutagen ausgesetzen Zellen so gering, dass das gesamte Zellmaterial über längere Zeit möglichst verlustfrei im Verfahren gehalten werden muss, um eine Kultur ausreichend lange lebensfähig zu erhalten, damit sie angepasste Wachstumsfaktoren ausbilden kann.

Es wurde nun überraschend gefunden, dass das Zentrifugieren entfällt, wenn ein Kulturmedium mit speziellen Eigenschaften verwendet wird, in dem viel mehr Zellen überleben, so dass der Verlust eines Teils der lebensfähigen Zellen infolge blossen Dekantierens unerheblich ist.

Durch den Verzicht auf das Zentrifugieren wird die Kontaminationsgefahr und die Gefahr einer mechanischen Schädigung der Zellen wesentlich vermindert, da der Transfer ins Zentrifugenröhrchen und das Zentrifugieren selbst entfällt. Ausserdem wird die Verweilzeit ausserhalb des Kulturraums, der gewöhnlich auf 37°C gehalten wird, stark verkürzt. Wesentliches Merkmal eines solchen angepassten Kulturmediums ist, dass es keine inhibierenden Stoffe resp. nicht notwendige Vorläufer inhibierender Stoffe enthält, und dass die Menge an essentiellen Stoffen, beispielsweise Aminosäuren, ausgewogen auf die Bedürfnisse einer speziellen Zelle abgestimmt sind.

Für CHO-Zellen haben sich beispielsweise Glutaminsäure sowie Mangan als stark inhibierend erwiesen. Ferner sind Asparaginsäure, Alanin, Cystin sowie die meisten Spurenelemente ausser Eisen, beispielsweise Kobalt, Vanadium, Selen, etc., unnötig bis schwach inhibierend. Diese Stoffe sollten deshalb in einem Kulturmedium für CHO-Zellen nicht vorhanden sein.

Eine günstige Zusammensetzung der Aminosäuren enthält pro Liter Kulturmedium die folgenden Mengen in mg, wobei gewisse Abweichungen, üblicherweise im Rahmen von ± 20 relativen Gew.-%, in Ausnahmefällen sogar bis 30 relativen Gew.-% und mehr, zulässig sind:

| | |
|---|---|
| L-Arg HCl | 188,33 |
| L-Asn H₂O | 127,42 |
| L-Cys HCl H₂O | 44,00 |
| L-Gln | 87,50 |
| Gly | 14,58 |
| L-His HCl H₂O | 46,29 |
| L-lle | 22,35 |
| L-Leu | 57,75 |
| L-Lys HCl | 70,42 |
| L-Met | 18,11 |
| L-Phe | 18,71 |
| L-Pro | 37,08 |
| L-Ser | 13,13 |
| L-Thr | 44,84 |
| L-Trp | 25,11 |
| L-Tyr | 23,72 |
| L-Val | 61,74 |

Beispiel: Herstellung serum- und proteinfrei kultivierbarer CHO-Zelle, die von der CHO-K1-Zelle abgeleitet sind.

Das serumhaltige Kulturmedium von CHO-K1-Zellen wurde durch frisches, serum- und proteinfreies Medium ersetzt, sobald die Zelldichte etwa zu 80 % flächendeckend war. Als Medium wurde das aus der Diss. ETH Nr. 9559 bekannte FMX-8 verwendet, welches die obengenannte, ausgewogene Aminosäurezusammensetzung aufweist und zudem die folgenden anorganischen Salze, Vitamine und anderen Zusätze enthält (die angegebenen Zahlen betreffen die Menge der jeweiligen Komponenten in mg pro Liter Kulturmedium):

| **Anorganische Salze** | | **Vitamine** | | **andere Komponenten** | |
|---|---|---|---|---|---|
| CaCl₂.2H₂O | 56,66 | Biotin | 0,01 | D-Glucose | 1700,00 |
| FeSO₄.7H₂O | 3,11 | Ca-pantothenat | 2,83 | Hypoxanthin | 10,00 |
| | | Cholinchlorid | 45,00 | | |
| KCl | 199,10 | Myo-inositol | 15,17 | Linolsäure | 0,07 |
| | | Folsäure | 2,33 | | |
| MgSO₄.7H₂O | 158,38 | Niacinamid | 1,98 | Thioctsäure | 0,18 |
| NaCl | 6955,00 | Pyridoxin HCl | 2,18 | Phenolrot | 2,67 |
| NaHCO₃ | 2100,00 | Ribofla vin | 0,40 | Putrescin | 0,27 |
| Na₂HPO₄ | 182,30 | Thiamin HCl | 1,46 | Na-pyruvat | 91,67 |
| ZnSO₄.7H₂O | 0,74 | Vitamin B₁₂ | 0,88 | Thymidin | 2,00 |

Auch bei diesen Komponenten sind Abweichungen in den oben angegebenen Konzentrationen tolerierbar.

Die Kulturen wurden in Gewebekulturbehältern gehalten und ohne Verdünnen der Zellen (cell splitting) während 1½ Monaten kultiviert. Da beim Zellwachstum nahezu alle Glucose in Lactat umgewandelt wurde, sank der pH-Wert in Kulturen bei mittleren bis hohen Dichten innerhalb von 2-3 Tagen unterhalb den Bereich, in dem das Wachstum unterstützt wird. Deshalb wurden in Intervallen von 2-3 Tagen 50 % des Ueberstands über der Kultur verworfen und durch frisches Medium ersetzt. Dieses Verfahren wurde verwendet, um den pH in einem physiologisch günstigen Bereich für das Zellwachstum zu halten und Beschränkungen der zugänglichen, notwendigen Nährstoffe durch Verbrauch oder Zerfall zu vermeiden.

Die erste Subkultivierung mit Aufteilung der Zellen (cell splitting) wurde 1½ Monate nach Inokulation mittels Verdünnung durchgeführt. Zu diesem Zeitpunkt waren die Zellen vollständig an die serumfreien Kulturbedingungen angepasst und wuchsen in Suspension, d.h. sie waren nicht mehr von wachstumsunterstützenden Substraten abhängig. Die Zellkonzentration wurde bei der ersten Subkultivierung dreimal verdünnt, und die zweite, die 10 Tage später erfolgte, bereits fünfmal. Dies zeigt die Zunahme in der Zellwachstumsrate. Die Frequenz der Subkultivierung stieg stetig an, bis zu einer 20-fachen Verdünnung einmal pro Woche nach etwa 10 Durchgängen. Dank der Kulturbedingungen war es möglich, schnell wachsende Zellen zu selektionieren.

Die derart erhaltenen Zellen können im gleichen Medium ohne Protein- oder Serumzusatz, beispielsweise in T-Flaschen, in Ruhe kultiviert werden. Sobald das Medium aufgebraucht ist, wird eine Subkultivierung durch Verdünnung in frischem Medium ohne Zentrifugieren der Zellen durchgeführt. Die Zelldichte wird konstant bei etwa 4-5 x 10⁴ Zellen pro ml gehalten. Das Kulturvolumen wird drei Tage nach dem Zellsplitting durch Zugabe frischen Mediums ohne Subkultivierung vergrössert. Es hat sich als günstig erwiesen, die Subkultivierung in einem Intervall von 7 Tagen durchzuführen.

Derart erhaltene Zellen sind am 5. August 1993 bei der European Collection of Animal Cell Cultures (ECACC) unter der Nummer 93080520 (CHO Messi) gemäss den Bestimmungen des Budapester Vertrags von 1977 hinterlegt worden. Die Chromosomenzahl dieser Zellen variiert im Bereich von 18 bis 20. Solche variablen Chromosomenzahlen werden insbesondere bei frisch- resp. neu selektionierten Zellkulturen häufig beobachtet.

Die erfindungsgemässen Zellen eignen sich dank ihrer Serumunabhängigkeit, die eine geringere Kontaminationsgefahr bewirkt sowie hohe Konzentrationen und gute Durchmischung im Bioreaktor bei reproduzierbaren, voll definierten Kulturbedingungen ermöglicht, und dank ihrer guten Wachstumsgeschwindigkeit ausgezeichnet als Wirtszellen für die gen- resp. biotechnologische Herstellung von Produkten, insbesondere solchen Produkten, die aufgrund struktureller Anforderungen resp. posttranslatorischer Modifikationen in eukaryontischen Zellen hergestellt werden müssen. Dank der oben genannten Vorteile lassen sich die Produktionskosten vieler gen- resp. biotechnologisch herstellbarer resp. herzustellender Produkte senken.

Es ist dem Fachmann selbstverständlich klar, das die erfindungsgemässen Zellen mit den gleichen Verfahren gentechnologisch verändert werden können, wie bekannte Wirtszellen.

## Patentansprüche

1. Eukaryontische Zelle mit im wesentlichen runder Morphologie, die substratunabhängig ist und in einem serum- und proteinfreien Medium gezüchtet werden kann, dadurch gekennzeichnet, dass sie in einem Medium kultivierbar ist, welches keine lebenswichtigen Stoffe enthält, die die ursprüngliche Zelle in ihrer natürlichen Umgebung selbst produziert.

2. Zelle nach Anspruch 1, dadurch gekennzeichnet, dass sie eine CHO-Zelle ist.

3. Zelle nach Anspruch 1 oder 2, dadurch gekennzeichnet, dass sie in einem hypoxanthin-, glycin- und thymidinfreien Medium kultivierbar ist.

4. Zelle nach einem der Ansprüche 1-3, dadurch gekennzeichnet, dass sie das DHFR-Gen enthält.

5. Verfahren zur Selektion einer Zelle nach einem der Ansprüche 1-4 durch Ersatz des serum- und proteinhaltigen Mediums durch ein serum- und proteinfreies Medium, dadurch gekennzeichnet, dass das serum- und proteinfreie Medium keine wachstumsinhibierenden und keine mutagenen Stoffe enthält.

6. Verfahren nach Anspruch 5, dadurch gekennzeichnet, dass das Medium ohne Zusatz von Mangan und Glutaminsäure hergestellt wird.

7. Verfahren nach Anspruch 5 oder 6, dadurch gekennzeichnet, dass die Zellkultur nie zentrifugiert wird.

8. Verfahren nach einem der Ansprüche 5 bis 7, dadurch gekennzeichnet, dass das Medium die Aminosäuren pro Liter Kulturmedium in den folgenden Mengen in mg, wobei bis zu 30 % relative Abweichung zulässig ist, enthält:
| | |
|---|---|
| L-Arg HCl | 188,33 |
| L-Asn H₂O | 127,42 |
| L-Cys HCl H₂O | 44,00 |
| L-Gln | 87,50 |
| Gly | 14,58 |
| L-His HCl H₂O | 46,29 |
| L-lle | 22,35 |
| L-Leu | 57,75 |
| L-Lys HCl | 70,42 |
| L-Met | 18,11 |
| L-Phe | 18,71 |
| L-Pro | 37,08 |
| L-Ser | 13,13 |
| L-Thr | 44,84 |
| L-Trp | 25,11 |
| L-Tyr | 23,72 |
| L-Val | 61,74 |

9. Verfahren nach einem der Ansprüche 5 bis 8, dadurch gekennzeichnet, dass ein Kulturmedium verwendet wird, welches Glucose in einer Menge von 1700 mg/Liter enthält, wobei bis zu 30 relativen Gewichts-% Abweichung tolerierbar ist.

10. Verwendung der Zellen gemäss einem der Ansprüche 1-4 als Wirtszellen in der Gen- und Biotechnologie.
